# EUROPÄISCHE PATENTSCHRIFT

(11) **EP 1 384 997 B1**
(45) Veröffentlichungstag und Bekanntmachung des Hinweises auf die Patenterteilung: **29.09.2004**
(21) Anmeldenummer: 02016627.8
(22) Anmeldetag: 25.07.2002
(51) Int. Cl.: G01N 33/20, G01N 27/90, B22D 11/18

(54) **Verfahren und Vorrichtung zur Auswertung von Wirbelstrom-Messsignalen**
Method and device for processing eddy current signals
Méthode et dispositif pour traiter des signaux de courant de foucault

(43) Veröffentlichungstag der Anmeldung: 28.01.2004
(73) Patentinhaber: AMEPA ANGEWANDTE MESSTECHNIK UND PROZESSAUTOMATISIERUNG GMBH, D-52070 Aachen (DE)
(72) Erfinder: Julius, Edmund, Dr., 52070 Aachen (DE)
(74) Vertreter: Bauer, Dirk, Dipl.-Ing. Dipl.-Kfm.

(56) Entgegenhaltungen:
- AT-B- 379 534
- DE-A- 1 598 606
- PATENT ABSTRACTS OF JAPAN vol. 008, no. 019 (C-207), 26. Januar 1984 (1984-01-26) & JP 58 185706 A (SHIN NIPPON SEITETSU KK), 29. Oktober 1983 (1983-10-29)
- PATENT ABSTRACTS OF JAPAN vol. 012, no. 266 (P-735), 26. Juli 1988 (1988-07-26) & JP 63 052053 A (HITACHI LTD), 5. März 1988 (1988-03-05)

## Beschreibung

Die Erfindung betrifft ein Verfahren zum Erfassen von Bestandteilen, die in einem strömenden, elektrisch leitenden Fluid mitfließen und sich in der elektrischen Leitfähigkeit von dem Fluid unterscheiden, anhand von Störungen eines elektromagnetischen Feldes an einer von dem Fluid durchströmten Messstelle.

Dabei kann das Fluid eine elektrisch leitfähige Schmelze sein, die durch einen Kanal aus einem metallurgischen Gefäß ausfließt. Die Bestandteile sind dann typischerweise Gase oder Schlacken. Unter zusammenhängenden Bestandteilen sind Bereiche von Bestandteilen zu verstehen, die insbesondere in Flussrichtung der Schmelze ausgedehnt sind wie z. B. Fäden und deren Ausdehnung in Strömungsrichtung typischerweise sehr viel größer ist, als der Kanaldurchmesser. Unter diskreten Bestandteilen sind unzusammenhängende Bereiche von Bestandteilen oder Partikel zu verstehen, deren Ausdehnung in Bewegungsrichtung typischerweise kleiner als der Kanaldurchmesser ist.

In einem solchen Verfahren wird dann die Störung eines die strömende Schmelze zumindest teilweise durchdringenden elektromagnetischen Feldes an einer von der Schmelze durchströmten Messstelle, welches von mindestens einer von einem Wechselstrom durchflossenen Sendespule generiert wird, ausgewertet. In solchen Verfahren eingesetzte Vorrichtungen weisen neben der Sendespule ein Messelement zum Messen von Störungen des Feldes an der Messstelle und eine Auswerteeinrichtung auf, mittels derer nichtmetallische Bestandteile wie Gase oder Schlacke anhand einer Störung des Feldes detektierbar sind.

Beim Umfüllen und Ausgießen von Metallschmelzen aus einem metallurgischen Gefäß (zum Beispiel einem Konverter, einer Pfanne oder einem Verteiler) ist man bestrebt, die auf der Metalloberfläche schwimmende Schlacke - nichtmetallische Phasenbestandteile - nicht in das nächste Gefäß zu übertragen. Die gattungsgemäßen Verfahren und Vorrichtungen dienen der Überwachung der ausfließenden Schmelze, damit beim Detektieren von Schlacke Maßnahmen ergriffen werden können, um den Übergang von Schlacke zu unterbinden. Diese Maßnahmen können in der Generierung eines Warnsignals oder in der automatischen

Beendigung des Umfüllprozesses bestehen oder auf eine Beeinflussung der Strömung zielen. Maßnahmen zur Beeinflussung der Strömung sind beispielsweise die Verringerung des Ausflussquerschnitts oder das Einblasen von Gasen, typischerweise Argon oder Stickstoff, in den Ausflussbereich um die Ausbildung eines Strömungswirbels zu verhindern. Die Messstelle ist typischerweise oberhalb eines den Ausfluss steuernden Regelorgans angeordnet.

In solchen Verfahren und Vorrichtungen wird mittels einer von Wechselstrom durchflossenen Sendespule an einer Messstelle im Ausflusskanal ein magnetisches Wechselfeld aufgebaut. Dieses Feld erzeugt in der strömenden Schmelze eine Spannung, die ihrerseits in der elektrisch leitenden Schmelze einen Strom (Wirbelstrom) erzeugt. Dieser Strom wiederum erzeugt ein magnetisches Wechselfeld, das mit einem Messelement gemessen werden kann.

Enthält die ausströmende Schmelze Bestandteile, die eine geringere elektrische Leitfähigkeit als das Metall aufweisen, so ändert sich die Stromverteilung in der Schmelze und damit die Feldstärke des magnetischen Wechselfeldes. Durch Messung der Änderung der magnetischen Feldstärke an der Messstelle werden mitgeführte nichtmetallische Bestandteile erfasst. Erreicht die aufsummierte Änderung der Feldstärke eine Grenzamplitude, wird ein Warnoder/und ein Steuersignal ausgelöst.

Ein gattungsgemäßes Verfahren und eine entsprechende Vorrichtung ist in der DE 31 42 681 A1 beschrieben. Hier ist vorgeschlagen, Änderungen des elektromagnetischen Feldes an der Messstelle mittels der in einer Empfangsspule induzierten Spannung zu messen, die ebenso wie die Sendespule konzentrisch um den Ausflusskanal eines metallurgischen Gefäßes angeordnet ist.

In der DE 34 39 369 A1 und der DE 37 22 795 A1 werden Verbesserungen dieses Verfahren dargestellt. Einerseits wird vorgeschlagen, die Sendespule mit verschiedenen, einander überlagernden Frequenzen zu beaufschlagen. Aus der Reaktion in der Empfangsspule wird dann ein hoch differenziertes Bild der strömenden Schmelze erkennbar, so dass in dieser bereits ein sehr geringer Schlackenanteil detektiert werden kann. Andererseits wird vorgeschlagen, zur Verminderung der Signaldrift - also einer Verfälschung der an der Messstelle gemessenen magnetischen Feldstärke - aufgrund von Temperaturänderungen der ferromagnetischen Bodenplatte eines metallurgischen Gefäßes die Sende- und Empfangsspulen in einem nicht magnetischen Gehäuse anzuordnen.

Allgemein bekannt ist darüber hinaus, die Signaldrift durch Messen der Spulentemperatur und Korrektur der Messwerte mindestens teilweise zu kompensieren. Befinden sich allerdings ferromagnetische Metallteile in der Nähe der Spulen, so ist der Zusammenhang zwischen Temperatur und Drift der Messsignale nichtlinear, so dass der Einfluss der Temperatur auf die Signale nicht vollständig eliminiert werden kann. Die AT 379 534B offenbart ein Verfahren, bei dem das Messsignal mittels eines Hochpassfilters von niederfrequenten Anteilen befreit wird. Trotz der bereits erreichten Verbesserungen wird auch mit den bekannten gattungsgemäßen Verfahren und Vorrichtungen gegen Ende des Entleerungsvorganges regelmäßig eine Restmenge an Schlacke in das nächste Gefäß übertragen. Steigende Anforderungen an den Reinheitsgrad des Endproduktes sind mit den bekannten Verfahren und Vorrichtungen daher häufig nicht zu erfüllen.

Die Ursache dieser geringen Restmenge ist einerseits in der technisch bedingten Nachweisgrenze der verwendeten gattungsgemäßen Vorrichtungen, andererseits in einem als "kontinuierliches Einmischen" bekannten Vorgang zu suchen: Gegen Ende des Entleerungsvorganges beim Ausfließen einer Flüssigkeit aus einem Gefäß kann eine so genannte Wirbelsenke entstehen. Die aufgrund ihrer geringeren Dichte auf der Oberfläche der metallischen Schmelze "schwimmende" Schlacke wird durch einen solchen Wirbel als "Faden" in den Ausflusskanal eingezogen, wobei dessen Querschnitt und damit der Masseanteil der Schlacke in der Schmelze von nahezu Null kontinuierlich ansteigt. Solange der Masseanteil der Schlacke in der Schmelze unterhalb der Grenzamplitude der gattungsgemäßen Vorrichtung liegt, wird er von dieser nicht detektiert und die Schlacke fließt unbemerkt mit. Die Grenzamplitude der gattungsgemäßen Vorrichtungen kann jedoch nicht beliebig gesenkt werden, da in der Signaltechnik allgemein als "Rauschen" bezeichnete Störsignale und insbesondere Temperaturdriften die Messsignale überlagern. Durch diese nicht vermeidbaren Fehlerquellen ist für die gattungsgemäßen Verfahren und Vorrichtungen eine quasi "natürliche" Nachweisgrenze definiert, die nicht unterschritten werden kann.

Nach der herrschenden Lehrmeinung ist der bekannte Vorgang des "kontinuierlichen Einmischens" von Schlacke beim Entstehen einer Wirbelsenke dadurch gekennzeichnet, dass die Schlacke von Beginn des Einmischvorgangs an in Form eines "Schlackenfadens" mitgeführt wird, dessen Querschnitt mehr oder weniger kontinuierlich ansteigt. Bei der spektralen Analyse der Messwerte der gattungsgemäß bekannten Vorrichtungen wurden pulsförmige Störungen des Feldes an der Messstelle beobachtet. Die Impulsform dieser Störungen entspricht der von diskreten, das elektromagnetische Feld der Sendespule passierenden elektrisch nichtleitenden Konzentrationen in der Schmelze.

Durch gezielte Beobachtung dieser nach herrschender Lehrmeinung als "Rauschen" zu vernachlässigenden Störungen wurde nachgewiesen, dass dem kontinuierlichen Einsaugen eines dünnen Fadens häufig das Einsaugen kleiner diskreter Mengen vorausgeht oder dass auch der Faden mehrfach unterbrochen wird.

Der Erfindung liegt die Aufgabe zugrunde, ein Verfahren und eine Vorrichtung zum Auswerten von Messsignalen eines elektromagnetischen Feldes, das in Wechselwirkung mit einem elektrisch leitenden Fluid steht, zum Detektieren von Bestandteilen in dem Fluid, die sich in der elektrischen Leitfähigkeit von dem Fluid unterscheiden, insbesondere zum Detektieren von mitgeführter Schlacke in einer strömenden metallischen Schmelze vorzuschlagen, mit dem die Restmenge der gegen Ende des Umfüllens aus einem metallurgischen Gefäß mitgeführten Schlacke gegenüber den bekannten Verfahren und Vorrichtungen deutlich gesenkt wird.

Insbesondere soll ein Verfahren vorgeschlagen werden, mit dem gleichzeitig sowohl sehr schwache, als auch sehr starke Messsignale, die aus einem elektromagnetischen Feld stammen, das in Wechselwirkung mit einer strömenden metallischen Schmelze steht, in der sich nicht-metallische Bestandteile befinden, und deren Anteil an der Schmelze sehr klein aber auch sehr groß sein kann und die diskret aber auch als in Bewegungsrichtung ausgedehnte Bereiche auftreten können, erfasst und ausgewertet werden können.

Andererseits kann das Gefäß auch nahezu ohne einen Ausflusswirbel auslaufen. Dann steigt der Volumenanteil des leichteren Stoffes nahezu schlagartig an. Es stellt sich also die Aufgabe, Verfahren und Vorrichtungen vorzuschlagen, womit in einer ausfließendem Metallschmelze nichtmetallische Beimengungen, insbesondere Schlacke, mit größerer Empfindlichkeit erfasst werden können. Dabei sollen nicht nur geringere Mengen der Beimengungen erfasst werden, sondern insbesondere kleine diskrete Beimengungen sowie der Zeitpunkt des Auftretens detektiert werden.

Ausgehend von den gattungsbildenden Verfahren und Vorrichtungen wird die Aufgabe der Erfindung auf Basis dieser Erkenntnis dadurch gelöst, dass zusammenhängend mitfließende, in Strömungsrichtung ausgedehnte Bestandteile anhand von Störungen in einem ersten Kanal oberhalb einer unteren Grenzfrequenz f_{Gu} und gleichzeitig in dem Fluid verteilte diskrete Bestandteile oberhalb einer oberen Grenzfrequenz f_{Go} in einem zweiten Kanal erfasst werden.

Um den Einfluss von Störungen des Feldes durch insbesondere Temperaturänderungen zu vermindern, werden mitfließende nichtmetallische Bestandteile, insbesondere in Bewegungsrichtung ausgedehnte fadenförmige Beimengungen, anhand von Störungen des Feldes oberhalb einer unteren Hochpass Grenzfrequenz f_{Gu} erfasst. Bei dem Einwirbeln und Anwachsen von fadenförmigen Beimengungen handelt es sich um einen langsamen Vorgang im Sekunden- bis Minutenbereich. Die dadurch hervorgerufenen Störungen des Feldes können durch ein Hochpassfilter von den Störungen des Feldes durch Temperaturänderungen, zumindest teilweise, separiert werden. Die beiden Zeitbereiche überlagern sich jedoch und dadurch können sehr kleine Störungen des Feldes nicht eindeutig einem Zeitbereich zugeordnet werden.

Darüber hinaus werden in der Schmelze verteilte diskret mitfließende nichtmetallische Bestandteile, anhand von Störungen des Feldes oberhalb einer oberen Hochpass-Grenzfrequenz f_{Go} detektiert. Diskret mitfließende Bestandteile erzeugen pulsförmige Störungen des Feldes deren Breite um mehrere Zehnerpotenzen geringer ist, als durch Temperaturänderungen hervorgerufene Störungen und deutlich geringer als durch in Strömungsrichtung ausgedehnte Beimengungen hervorgerufene Störungen. Die durch diskret mitfließende Bestandteile erzeugten Störungen des Feldes können durch ein zweites Hochpassfilter mit einer Grenzfrequenz f_{Go} von den anderen Störungen nahezu vollständig getrennt und separat verstärkt werden. Dadurch können Konzentrationen von mitfließenden nichtmetallischen Bestandteilen erfasst werden, die um mehr als eine Zehnerpotenz unter denen bekannter Vorrichtungen liegen. Neben der Erfassung geringster Konzentrationen an Bestandteilen ist der Zeitpunkt und die Menge von mitfließenden diskreten Beimengungen ein Indiz für das bevorstehende Mitfließen größerer Mengen an nichtmetallischen Bestandteilen. Die Kenntnis der Menge und des Zeitpunktes mitfließender Bestandteile ermöglichen ein frühzeitiges Auslösen von strömungsbeeinflussenden Maßnahmen, so dass die mitfließende Menge an Bestandteilen gegenüber heutigen Verfahren drastisch reduziert wird.

Besonders vorteilhaft wird der Signalverlauf des zweiten Kanals von dem Signalverlauf des ersten Kanals subtrahiert. Das resultierende Differenzsignal kann dann verwendet werden, um in Strömungsrichtung ausgedehnte Bestandteile in der Schmelze zu erfassen.

Beim Überwachen von metallischen Schmelzen beim Ausfließen aus metallurgischen Gefäßen hat sich für die untere Hochpassgrenzfrequenz das Produkt aus Grenzfrequenz und Strömungsgeschwindigkeit an der Messstelle zwischen 0,001 m/s² bis 0,01 m/s² beziehungsweise für die obere Hochpassgrenzfrequenz von 0,1 m/s² bis 10m/s² als günstig erwiesen.

Die Auswerteeinrichtung einer erfindungsgemäßen Vorrichtung wird hierzu in einem ersten Kanal mit einem entsprechenden Hochpass-Filterelement der Grenzfrequenz f_{Gu} ausgestattet. Mit diesem Kanal kann das Mitfließen von nichtmetallischen, insbesondere in Strömungsrichtung ausgedehnten, fadenförmigen Beimengungen bei gleichzeitiger Verminderung der Störungen durch Temperaturänderungen erfasst werden. In einem zweiten Kanal wird die Auswerteeinrichtung mit einem Hochpass-Filterelement der Grenzfrequenz f_{Go} ausgestattet. So können diskret mitfließende Bestandteile getrennt erfasst und weiterverarbeitet werden. Die Vorrichtung erlaubt so z. B. auch den gleichzeitigen Nachweis von in Strömungsrichtung ausgedehnten Bestandteilen an Schlacke und diskret mitfließenden Schlackebestandteilen und/oder Gasblasen. Mit dieser Erfindung werden insbesondere größere zusammenhängende Verteilungen der Bestandteile und kleinere diskret auftretende Bestandteile in dem Fluid getrennt erfasst und ausgewertet.

Als Messelement werden in den erfindungsgemäßen Verfahren und Vorrichtungen bevorzugt Empfangsspulen eingesetzt, in denen ein von einer Sendespule generiertes magnetisches Wechselfeld wieder eine Wechselspannung induziert. Eine Störung des Feldes kann dann als Störung der in der Empfangsspule induzierten Wechselspannung gemessen werden. Grundsätzlich ist es möglich, die Sendespule zugleich als Empfangsspule zu nutzen, da auch in dieser die induktive Wirkung des elektromagnetischen Feldes messbar ist. Auf diese Weise kann eine erfindungsgemäße Vorrichtung vorteilhaft wiederum besonders platzsparend gestaltet werden.

Die in der Empfangsspule induzierte Spannung setzt sich aus zwei Komponenten zusammen. Durch das elektromagnetische Feld der Sendespule wird in der Empfangsspule eine Spannung Uₒ induziert. Diese ist eine Funktion des Sendestromes, der Frequenz und der Gegeninduktivität zwischen Sende- und Empfangsspule. Durch das elektromagnetische Feld der Sendespule wird in der strömenden Schmelze eine Spannung induziert, die proportional zu dem Sendestrom, der Frequenz und der Gegeninduktivität zwischen Sendespule und Schmelze ist. Diese Spannung ruft ihrerseits Wirbelströme in der Schmelze hervor, die wiederum ein elektromagnetisches Feld erzeugen, das in der Empfangsspule eine Spannung dU induziert, die proportional zu der Größe der Wirbelströme, der Frequenz und der Gegeninduktivität zwischen Schmelze und Empfangsspule ist. Die Messempfindlichkeit dieser Vorrichtung hat ihre Grenze dort, wo eine Spannungsänderung dU in der Spannung Uₒ noch eindeutig erkannt wird. Je größer das Verhältnis dU/Uₒ ist, um so größer ist die Messempfindlichkeit der Vorrichtung.

Bei der Detektion von Schlacke kann es sinnvoll sein, den Entleerungsvorgang des metallurgischen Gefäßes schon bei den geringsten Mengen von mitfließender Schlacke zu beenden. Dann ist es ausreichend, wenn die Auswerteeinheit nur ein Filterelement aufweist, mit dem diskret mitfließende Bestandteile nachgewiesen werden.

Abhängig von den Qualitätsanforderungen der Nutzer der Vorrichtung dürfen mehr oder weniger nichtmetallische Bestandteile mit der Schmelze ausfließen. Vorzugsweise hat die Vorrichtung daher ein Element, das die erfassten Messwerte aufsummiert und damit eine Größe generiert, die der Menge der mitgeflossenen Bestandteile proportional ist und das beim Überschreiten eines festgelegten Grenzwertes ein Signal zur Ansteuerung einer strömungsbeeinflussenden Einrichtung, z. B. eine Verschlusseinrichtung, liefert.

In der erfindungsgemäßen Vorrichtung ist die Sendespule und/oder die Empfangsspule vorzugsweise von der Schmelze durchströmbar, die Wicklungen der betreffenden Sendespule sind mindestens teilweise um die strömende Schmelze herum angeordnet.

In einer bevorzugten Ausführungsform werden die Sende- und/oder Empfangsspulen in einem metallischen, mindestens teilweise nicht ferromagnetischen, Gehäuse angeordnet. Dieses Gehäuse dient als Träger und Schutz der Spulen gegen mechanische und thermische Belastungen. Damit die elektromagnetischen Felder einen Abschnitt des Gehäuses durchdringen können, muss dieser aus einem nicht ferromagnetischen Material bestehen.

Besonders bevorzugt werden jeweils einander zugeordnete Sende- und Empfangsspulen in einem gemeinsamen metallischen Gehäuse angeordnet. Die so gebildete bauliche Einheit aus Sende- und Empfangsspule erleichtert insbesondere den Austausch sowie die Nachrüstung. Zu diesem Zweck können die für den jeweiligen Einsatz spezifisch angepassten Spulen in den speziell ausgestalteten metallischen Gehäusen untergebracht sein, die einen für den rauen Betrieb des Kunden einfachen Austausch ermöglichen. Darüber hinaus sind die Spulen gegen mechanische und thermische Beanspruchungen geschützt.

Eine weitere Verbesserung der Messempfindlichkeit der Vorrichtung kann erreicht werden, wenn die Sende- und die Empfangsspule axial beabstandet und durch eine metallische Wand von einander getrennt sind und entweder beide Spulen in einem gemeinsamen Gehäuse oder jede Spule in einem separaten Gehäuse angeordnet ist bzw. sind, wobei das oder die Gehäuse aus metallischem Material besteht bzw. bestehen und das metallische Material zumindest abschnittsweise nicht ferromagnetisch ist. Liegen Sende- und Empfangsspule dicht beieinander, ist die Gegeninduktivität zwischen ihnen groß und demzufolge auch die Spannung Uₒ. Kann die Gegeninduktivität zwischen Sende- und Empfangsspule verringert werden, ohne dass die Gegeninduktivität zwischen Sendespule und Schmelze und die zwischen Schmelze und Empfangsspule in gleichem Masse verringert wird, kann die Messempfindlichkeit gesteigert werden. Dies wird mit der vorgeschlagenen Vorrichtung erreicht. Durch einen Abstand zwischen Sende- und Empfangsspule wird die Gegeninduktivität zwischen beiden Spulen deutlicher verringert, als die Gegeninduktivitäten zwischen den Spulen und der strömenden Schmelze, so dass sich das Verhältnis dU/Uₒ vergrößert. Dieser Effekt wird weiter durch eine metallische Trennwand zwischen Sende- und Empfangsspule verstärkt. Dabei sollte der Abstand d kleiner sein, als die Differenz zwischen dem inneren Radius des Spulengehäuses und dem Innenradius des von der Schmelze durchströmten Kanals.

Sende- und/oder Empfangsspulen einer erfindungsgemäßen Vorrichtung können vorteilhaft in einen Abschnitt eines meist aus Keramik bestehenden Ausflusskanals eines metallurgischen Gefäßes integriert werden. Die Messstelle - und damit der Ort, nach dessen Zustand über die Fortsetzung des Gießvorganges entschieden wird - ist dann besonders nahe am Ausfluss des metallurgischen Gefäßes angeordnet.

Die erfindungsgemäßen Verfahren und Vorrichtungen eignen sich in besonderem Maße zur weiteren Auswertung der in den Messstellen ermittelten Signale, insbesondere zur Klassifizierung in Mustern oder zur statistischen Korrelation mit weiteren Informationen, die einen Einfluss auf das Mitfließen von Verunreinigungen haben können. Derartige Informationen können beispielsweise der aktuelle Restinhalt des metallurgischen Gefäßes, der Verschleißzustand des Ausflusskanals oder das Alter der keramischen Auskleidung sein. So können beispielsweise aus vorhergegangenen Gießvorgängen Vergleichsgrößen und Schätzungen für zukünftige Entleerungen oder Hinweise zur Auslösung von Warn- oder Steuersignalen beim Erkennen typischer, dem Mitfluss von Verunreinigungen vorausgehender Muster abgeleitet werden.

Zur Erläuterung der Erfindung sind nachfolgend in Zeichnungen Ausführungsbeispiele dargestellt. Hierbei sind gleichartige Elemente in verschiedenen Ausführungsformen mit gleicher Nummer und unterschiedlichen Buchstaben gekennzeichnet. Es zeigen
- Fig. 1: einen Längsschnitt durch den Ausflusskanal einer Gießpfanne mit einer erfindungsgemäßen Vorrichtung,
- Fig. 2: eine erste Spulenanordnung für eine erfindungsgemäße Vorrichtung,
- Fig. 3: einen vergrößerten Längsschnitt durch den Ausflusskanal nach Fig. 1
- Fig. 4: einen Schaltplan der erfindungsgemäßen Vorrichtung,
- Fig. 5: die Filtercharakteristik der erfindungsgemäßen Vorrichtung
- Fig. 6: eine zweite Spulenanordnung für eine erfindungsgemäße Vorrichtung,

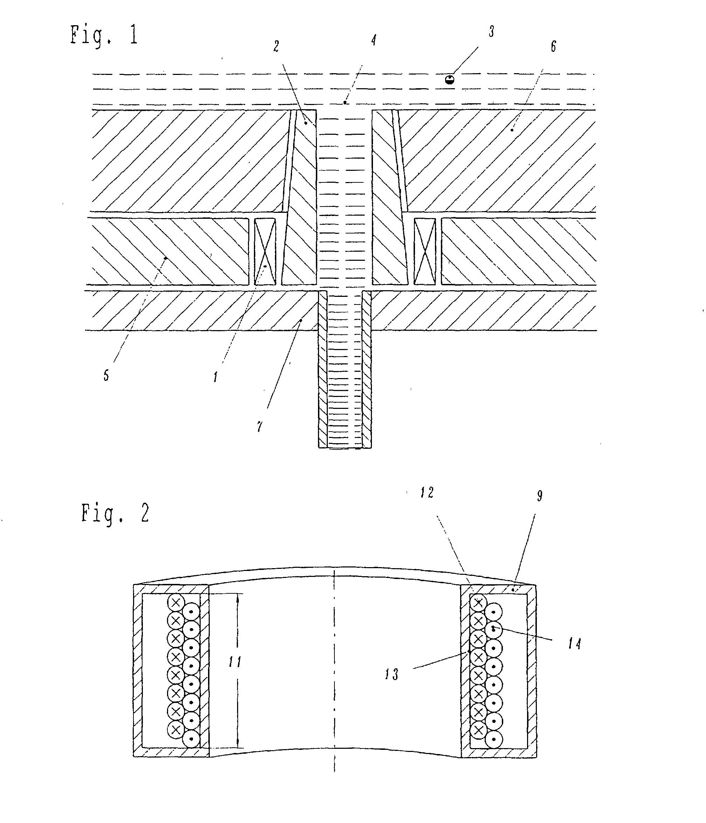

Figur 1 zeigt die Spulenanordnung 1 einer erfindungsgemäßen Vorrichtung am keramischen Ausflusskanal 2 im Bodenbereich 3 einer nicht weiter dargestellten Gießpfanne, durch die metallische Schmelze 4 ausfließt. Die Ausflussgeschwindigkeit am Messort liegt bei 2 m/s.

Die Gießpfanne weist im Bodenbereich eine stählerne Tragkonstruktion 5 und eine keramische Auskleidung 6 auf, die die stählerne Tragkonstruktion 5 vor Beschädigung durch die metallische Schmelze 4 schützt. Der keramische Ausflusskanal 2 durchdringt die Auskleidung 6 und die Tragkonstruktion 5 und ist unterhalb der Tragkonstruktion 5 durch einen Plattenschieber 7 in nicht näher dargestellter Weise verschließbar. Die Spulenanordnung 1 ist zwischen Auskleidung 6 und Plattenschieber 7 um den Ausflusskanal 2 herum in die Tragkonstruktion 5 eingesetzt.

Die in Figur 2 separat dargestellte Spulenanordnung ist torusförmig mit einem rechteckigen, in Richtung der Torusachse 7 gestreckten Gehäuse 9 gestaltet. Das torusförmige Gehäuse 9 wird anwendungsspezifisch an die den Ausflussbereich des metallurgischen Gefäßes bestimmenden Bauteile, wie Dicke der Tragkonstruktion und/oder Durchmesser des keramischen Ausflusskanals angepasst. Der Innendurchmesser des Gehäuses liegt beispielsweise bei 300 mm, die Wandstärke des austenitischen Gehäuses bei 1 mm. Innerhalb des Gehäuses 9 ist an dessen Innenseite 13 über seine gesamte Höhe 11 eine Sendespule 12 und eine Empfangsspule 14 angeordnet. Die Windungszahl der Spulen ist 25. Die Sendespule ist mit einem hier nicht gezeigten Wechselstromgenerator, der die Sendespule mit einem Wechselstrom von z. B. 100 mA und einer Frequenz von 100 Hz speist, und die Empfangsspule mit dem Eingang des Demodulator 25 verbunden.

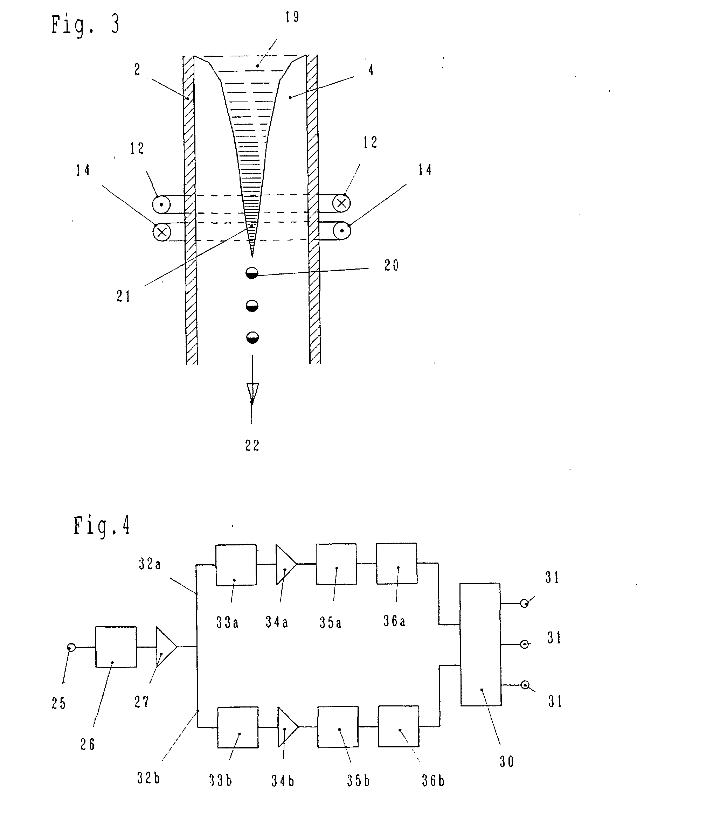

In Figur 3 ist die Funktionsweise der erfindungsgemäßen Vorrichtung schematisch dargestellt: die metallische Schmelze 4 fließt durch den keramischen Ausflusskanal 2 aus dem metallurgischen Gefäß. Gegen Ende des Ausflussvorganges wird zunehmend Schlacke 19 in den Ausflusskanal 2 mitgeführt. Die Schlacke 19 kann wie dargestellt dabei zuerst in diskreten Mengen 20 und erst später in einem kontinuierlichen Faden 21 eingezogen werden, dessen Massenanteil an der Schmelze 4 kontinuierlich ansteigt.

Die Sendespule 12 erzeugt in der ausströmenden metallischen Schmelze 4 entsprechend der anliegenden, nicht näher dargestellten Wechselspannung ein gleichfalls nicht näher dargestelltes magnetisches Wechselfeld, dessen Feldlinien in der Schmelze 4 in Höhe der Sendespule in Strömungsrichtung 22 verlaufen. Das magnetische Wechselfeld erzeugt in der metallischen Schmelze sogenannte Wirbelströme, die ihrerseits in der Empfangsspule 14 eine nicht näher dargestellte Spannung erzeugen, die an dieser zur Detektion von Schlacke abgegriffen wird.

Fig. 4 veranschaulicht die Signalverarbeitung. Die Spannung aus der Empfangsspule 14 wird auf den Eingang 25 geführt, in einem Messwandler 26 demoduliert und über einen Verstärker 27 geführt. Das verstärkte Signal wird in einem ersten Signalpfad 32a über ein erstes Filter 33a geführt und in einem Verstärker 34a verstärkt, in einem Summationselement 35a aufsummiert und in einem Amplitudenfilter 36a mit einer hier nicht dargestellten Grenzamplitude verglichen. Sowohl das Signal als auch ein zweites Signal bei Überschreiten der Grenzamplitude werden dem Auswertelement 30 zugeführt. Das verstärkte Signal wird in einem zweiten Signalpfad 32b über ein zweites Filter 33b geführt und gleichfalls in einem Verstärker 34 b verstärkt und in einem Summationselement 35b aufsummiert und in einem Amplitudenfilter 36b mit einer hier nicht dargestellten Grenzamplitude verglichen. Sowohl das Signal, als auch ein zweites Signal bei Überschreiten der Grenzamplitude werden dem Auswerteelement 30 zugeführt, das wiederum an den Ausgängen 31 Warn- und Steuersignale erzeugt.

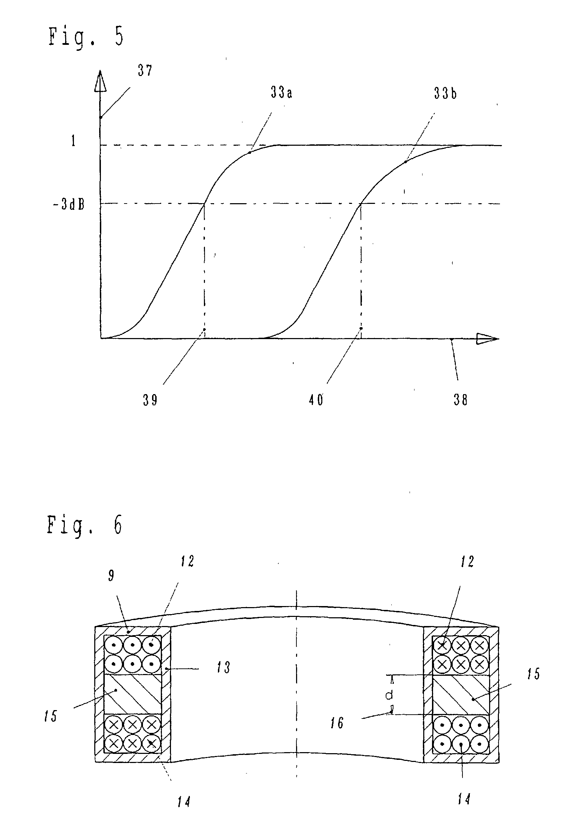

Die Übertragungscharakteristik 37 der Filter 33a und 33b ist in Figur 5 in einem gemeinsamen Diagramm als Funktion der Frequenz 38 dargestellt. Der erste Filter 33a weist eine Grenzfrequenz 39 bei 0,001 Hz auf. Der zweite Filter 33b weist eine Grenzfrequenz 40 bei 5 Hz auf. Die Grenzfrequenzen 39 und 40 bezeichnen diejenigen Frequenzwerte, unterhalb derer das jeweilige Eingangssignal um mehr als 3 dB gedämpft wird. Das Signal am Ausgang des ersten Filters 33a wird dann im Wesentlichen von größeren zusammenhängenden (fadenförmigen), das Signal am Ausgang des zweiten Filters 33b von diskret mitgeführten Schlackenbestandteilen erzeugt.

Fig. 6 zeigt eine alternative Ausgestaltung der Spulenanordnung nach Fig. 2. In dem Gehäuse 9 sind die Sendespule 12 und die Empfangsspule 14 mit einem Abstand 16 untergebracht und durch eine metallische Wand 15 getrennt. Das elektromagnetische Feld der Sendespule am Ort der Empfangsspule ist durch den Abstand der Spulen schwächer als bei unmittelbar aneinander liegenden Spulen und wird durch die Dämpfungswirkung der metallischen Wand weiter verringert. Die Sendespule ist mit einem hier nicht gezeigten Wechselstromgenerator und die Empfangsspule mit dem Eingang des Demodulator 25 verbunden.

## Patentansprüche

1. Verfahren zum Erfassen von Bestandteilen, die in einem strömenden, elektrisch leitenden Fluid mitfließen und sich in der elektrischen Leitfähigkeit von dem Fluid unterscheiden, anhand von Störungen eines elektromagnetischen Feldes an einer von dem Fluid durchströmten Messstelle, wobei zusammenhängend mitfließende, in Strömungsrichtung ausgedehnte Bestandteile anhand von Störungen in einem ersten Kanal oberhalb einer unteren Grenzfrequenz f_{Gu} und **dadurch gekennzeichnet, dass** gleichzeitig in dem Fluid verteilte diskrete Bestandteile oberhalb einer oberen Grenzfrequenz f_{Go} in einem zweiten Kanal erfasst werden.

2. Verfahren nach dem vorgenannten Anspruch, ***dadurch gekennzeichnet, dass*** in einer strömenden metallischen Schmelze mitfließende nichtmetallische Bestandteile erfasst werden, wobei das elektromagnetische Feld von mindestens einer von einem Wechselstrom durchflossenen Sendespule generiert wird.

3. Verfahren nach dem vorgenannten Anspruch, ***dadurch gekennzeichnet, dass*** die strömende metallische Schmelze eine aus einem metallurgischen Gefäß ausfließende Stahlschmelze ist und die nichtmetallischen Bestandteile Schlacken und/oder Gase sind.

4. Verfahren nach einem der vorgenannten Ansprüche, ***dadurch gekennzeichnet, dass*** ein Produkt aus der oberen Grenzfrequenz f_{Go} und einer Strömungsgeschwindigkeit v an der Messstelle zwischen 0,1 m/s² bis 10 m/s² liegt.

5. Verfahren nach einem der vorgenannten Ansprüche, **dadurch gekennzeichnet, dass** ein Produkt aus der unteren Grenzfrequenz f_{Gu} und einer Strömungsgeschwindigkeit v an der Messstelle zwischen 0,001 m/s² bis 0,01 m/s² liegt.

6. Verfahren nach einem der vorgenannten Ansprüche, ***dadurch gekennzeichnet, dass*** eine Störung des von einer Sendespule erzeugten elektromagnetischen Feldes anhand einer Störung einer in einer Empfangsspule induzierten Spannung detektiert wird.

7. Vorrichtung zum Erfassen von nichtmetallischen Bestandteilen in einer strömenden metallischen Schmelze mit mindestens einer von Wechselstrom durchflossenen Sendespule zum Generieren eines die strömende Schmelze zumindest teilweise durchdringenden elektromagnetischen Feldes, mit einem Messelement zum Messen von Störungen des Feldes an einer von der Schmelze durchströmten Messstelle und mit einer Auswerteeinrichtung, die ein erstes Filterelement aufweist, das Störungen oberhalb einer unteren Grenzfrequenz f_{Gu} in einen ersten Kanal leitet, mit dem in der Schmelze mitfließende, insbesondere in Strömungsrichtung ausgedehnte nichtmetallische Bestandteile erfassbar sind und **gekennzeichnet durch** ein zweites Filterelement, das Störungen des elektromagnetischen Feldes oberhalb einer oberen Grenzfrequenz f_{Go} in einen zweiten Kanal leitet, mit dem in der Schmelze verteilte, diskret mitfließende nichtmetallische Bestandteile erfassbar sind.

8. Vorrichtung nach dem vorgenannten Anspruch, ***gekennzeichnet durch*** mindestens in einem Kanal ein Summierelement, in dem die in dem Kanal erfassten Messwerte zu einem Summenwert aufsummiert werden, und ein Amplitudenfilter, das ein Signal auslöst, sofern der Summenwert eine Grenzamplitude überschreitet.

9. Vorrichtung nach einem der Ansprüche 7 oder 8, **dadurch gekennzeichnet, dass** das Produkt aus der oberen Grenzfrequenz f_{Go} und einer Strömungsgeschwindigkeit v an der Messstelle zwischen 0,1 m/s² und 10 m/s² liegt.

10. Vorrichtung nach einem der Ansprüche 7 bis 9, **dadurch gekennzeichnet, dass** das Produkt aus der unteren Grenzfrequenz f_{Gu} und der Strömungsgeschwindigkeit v an der Messstelle zwischen 0,001 m/s² und 0,01 m/s² liegt.

11. Vorrichtung nach einem der Ansprüche 7 bis 10, **dadurch gekennzeichnet, dass** ein Messelement eine Empfangsspule ist und dass Störungen des elektromagnetischen Feldes an der Messstelle anhand Störungen einer in der Empfangsspule induzierten Spannung detektierbar sind.

12. Vorrichtung nach dem vorgenannten Anspruch, ***dadurch gekennzeichnet, dass*** auch die Empfangsspule von der Schmelze durchströmbar ist.

13. Vorrichtung nach einem der Ansprüche 7 bis 12, **dadurch gekennzeichnet, dass** die Sendespule zugleich das Messelement ist.

14. Vorrichtung nach einem der Ansprüche 7 bis 13, **dadurch gekennzeichnet, dass** die Sende- und/oder die Empfangsspule jeweils einzeln in einem metallischen, mindestens teilweise nicht ferromagnetischen Gehäuse angeordnet sind.

15. Vorrichtung nach einem der Ansprüche 7 bis 14, **dadurch gekennzeichnet, dass** die Sende- und Empfangsspulen in einem gemeinsamen metallischen, mindestens teilweise nicht ferromagnetischen Gehäuse angeordnet sind.

16. Vorrichtung nach einem der Ansprüche 7 bis 15, **dadurch gekennzeichnet, dass** die Sendespule und die Empfangsspule axial beabstandet und durch eine metallische Wand von einander getrennt sind und entweder beide Spulen in einem gemeinsamen Gehäuse oder jede Spule in einem separaten Gehäuse angeordnet ist bzw. sind, wobei das oder die Gehäuse aus metallischem Material besteht bzw. bestehen und das metallische Material zumindest abschnittsweise nicht ferromagnetisch ist.

17. Vorrichtung nach einem der Ansprüche 7 bis 16, **dadurch gekennzeichnet, dass** die Sende- und Empfangsspulen in zumindest einen Abschnitt eines Ausflusskanals eines metallurgischen Gefäßes integriert sind.

18. Verfahren nach einem der Ansprüche 1 bis 6, ***dadurch gekennzeichnet, dass*** eine Vorrichtung nach einem der Ansprüche 7 bis 17 zur Auslösung eines Warnsignals und/oder eines Steuersignals zum Ansteuern einer Durchflussregeleinrichtung und/oder einer Einrichtung zur Modifikation der Strömung der metallischen Schmelze beim Erfassen diskreter und/oder zusammenhängender Verunreinigungen verwendet wird.

## Claims

1. A method for detecting components which are entrained in a flowing, electrically conductive fluid and differ in the electric conductivity from the fluid on the basis of disturbances of an electromagnetic field at a measuring point which is flowed through by the fluid, with contiguously entrained components which are expanded in the direction of flow are detected on the basis of disturbances in a first channel above a lower limit frequency f_{Gu}, **characterized in that** discrete components which are simultaneously distributed in the liquid are detected above an upper limit frequency f_{Go} in a second channel.

2. A method as claimed in the preceding claim, **characterized in that** in a flowing metallic melt entrained non-metallic components are detected, with the electromagnetic field being generated by at least one transmitting coil which is flowed through by alternating current.

3. A method as claimed in the preceding claim, **characterized in that** the flowing metallic melt is a steel melt flowing from a metallurgical vessel and the non-metallic components are slag and/or gases.

4. A method as claimed in one of the preceding claims, **characterized in that** a product of the upper limit frequency f_{Go} and a flow speed v at the measuring point lies between 0.1 m/s² to 10 m/s².

5. A method as claimed in one of the preceding claims, **characterized in that** a product of the lower limit frequency f_{Gu} and a flow speed v at the measuring point lies between 0.001 m/s² to 0.01 m/s².

6. A method as claimed in one of the preceding claims, **characterized in that** a disturbance of the electric field generated by the transmitting coil is detected by means of a disturbance of a voltage induced in a receiving coil.

7. An apparatus for detecting non-metallic components in a flowing metallic melt with at least one transmitting coil flowed through by alternating current for generating an electromagnetic field which penetrates the flowing melt at least partly, with a measuring element for measuring disturbances of the field at a measuring point flowed through by the melt and with an evaluation device having a first filter element which conducts disturbances above a first lower limit frequency f_{Gu} into a first channel with which non-metallic components which are entrained by the flow and which are expanded especially in the direction of flow can be detected, **characterized by** a second filter element which conducts disturbances of the electromagnetic above an upper limit frequency f_{Go} into a second channel with which non-metallic components which are distributed in the melt and are discretely entrained can be detected.

8. An apparatus as claimed in the preceding claim, **characterized by** at least one summation element in a channel, such that the measured variables detected in the channel are summed up into a cumulative value, and an amplitude filter which initiates a signal insofar as the cumulative value exceeds a limit amplitude.

9. An apparatus as claimed in one of the claims 7 or 8, **characterized in that** the product of the upper limit frequency f_{Go} and a flow speed v at the measuring point is between between 0.1 m/s² to 10 m/s².

10. An apparatus as claimed in one of the claims 7 to 9, **characterized in that** the product of the lower limit frequency f_{Gu} and a flow speed v at the measuring point is between between 0.001 m/s² to 0.01 m/s².

11. An apparatus as claimed in one of the claims 7 to 10, **characterized in that** a measuring element is a receiving coil and that disturbances of the electromagnetic field at the measuring point can be detected by way of disturbances of voltage induced in the receiving coil.

12. An apparatus as claimed in the preceding claim, **characterized in that** the receiving coil can also be flowed through by the melt.

13. An apparatus as claimed in one of the claims 7 to 12, **characterized in that** the transmitting coil is simultaneously the measuring element.

14. An apparatus as claimed in one of the claims 7 to 13, **characterized in that** the transmitting and/or receiving coil are each arranged individually in a metallic housing which at least in part is non-ferromagnetic.

15. An apparatus as claimed in one of the claims 7 to 14, **characterized in that** the transmitting and receiving coils are arranged in a common metallic housing which at least in part is non-ferromagnetic.

16. An apparatus as claimed in one of the claims 7 to 15, **characterized in that** the transmitting and receiving coils are axially spaced and are mutually separated by a metallic wall and either both coils are housed in a common housing or each coil is arranged in a separate housing, with the housing(s) consisting of a metallic material and the metallic material being non-ferromagnetic at least in sections.

17. An apparatus as claimed in one of the claims 7 to 16, **characterized in that** the transmitting and receiving coils are integrated in at least one section of an outflow channel of a metallurgical vessel.

18. A method as claimed in one of the claims 1 to 6, **characterized in that** an apparatus according to one of the claims 7 to 17 is used for initiating a warning signal and/or a control signal for triggering a flow control device and/or a device for modifying the flow of the metallic melt during the detection of discrete and/or contiguous impurities.

## Revendications

1. Procédé pour la détection de composants entraînés dans un fluide conducteur électrique qui s'écoule et ayant une conductivité électrique différente du fluide, à l'aide des perturbations d'un champ électromagnétique au niveau d'un point de mesure à travers lequel le fluide passe, dans lequel des composants entraînés cohérents et étendus dans le sens de l'écoulement sont détectés à l'aide de perturbations dans un premier canal au-dessus d'une fréquence limite inférieure f_{Gu} et **caractérisé en ce que** des composants discrets répartis dans le même temps dans le fluide sont détectés dans un second canal au-dessus d'une fréquence limite supérieure f_{Go}.

2. Procédé selon la revendication précédente, **caractérisé en ce que** des composants non métalliques entraînés dans une masse métallique en fusion qui s'écoule sont détectés, le champ électromagnétique étant produit par au moins une bobine émettrice parcourue par un courant alternatif.

3. Procédé selon la revendication précédente, **caractérisé en ce que** la masse métallique en fusion qui s'écoule est une masse d'acier en fusion s'écoulant d'un récipient métallurgique et les composants non métalliques sont des scories et (ou) des gaz.

4. Procédé selon l'une quelconque des revendications précédentes, **caractérisé en ce qu'**un produit de la fréquence limite supérieure f_{Go} et d'une vitesse d'écoulement v au niveau du point de mesure est compris entre 0,1 m/s² et 10 m/s².

5. Procédé selon l'une quelconque des revendications précédentes, **caractérisé en ce qu'**un produit de la fréquence limite inférieure f_{Gu} et d'une vitesse d'écoulement v au niveau du point de mesure est compris entre 0,001 m/s² et 0,01 m/s².

6. Procédé selon l'une quelconque des revendications précédentes, **caractérisé en ce qu'**une perturbation du champ électromagnétique produit par une bobine émettrice est détectée à l'aide d'une perturbation d'une tension induite dans une bobine réceptrice.

7. Dispositif pour la détection de composants non métalliques dans une masse de métal en fusion qui s'écoule, comprenant au moins une bobine émettrice parcourue par un courant alternatif pour produire un champ électromagnétique pénétrant au moins partiellement dans la masse en fusion qui s'écoule, un élément de mesure pour la mesure de perturbations du champ au niveau d'un point de mesure à travers lequel la masse en fusion passe et une installation d'interprétation qui comprend un premier élément de filtrage transmettant les perturbations situées au-dessus d'une fréquence limite inférieure f_{Gu} à un premier canal avec lequel les composants non métalliques entraînés dans la masse en fusion et notamment ceux étendus dans le sens de l'écoulement peuvent être détectés, et **caractérisé en ce qu'**il comprend un second élément de filtrage qui transmet les perturbations du champ électromagnétique situées au-dessus d'une fréquence limite supérieure f_{Go} à un second canal avec lequel les composants non métalliques discrets entraînés et répartis dans la masse en fusion peuvent être détectés.

8. Dispositif selon la revendication précédente, **caractérisé en ce qu'**il comprend au moins dans un canal un élément additionneur dans lequel les valeurs de mesure captées dans le canal sont additionnées pour donner une somme, et un filtre d'amplitude qui déclenche un signal dès que la valeur de la somme dépasse une amplitude limite.

9. Dispositif selon l'une quelconque des revendications 7 ou 8, **caractérisé en ce que** le produit de la fréquence limite supérieure f_{Go} et d'une vitesse d'écoulement v au niveau du point de mesure est compris entre 0,1 m/s² et 10 m/s².

10. Dispositif selon l'une quelconque des revendications 7 à 9, **caractérisé en ce que** le produit de la fréquence limite inférieure f_{Gu} et de la vitesse d'écoulement v au niveau du point de mesure est compris entre 0,001 m/s² et 0,01 m/s².

11. Dispositif selon l'une quelconque des revendications 7 à 10, **caractérisé en ce qu'**un élément de mesure est une bobine réceptrice et **en ce que** les perturbations du champ électromagnétique au niveau du point de mesure sont détectables à l'aide d'une tension induite dans la bobine réceptrice.

12. Dispositif selon la revendication précédente, **caractérisé en ce que** la masse en fusion peut passer à travers la bobine réceptrice.

13. Dispositif selon l'une quelconque des revendications 7 à 12, **caractérisée en ce que** la bobine émettrice est en même temps l'élément de mesure.

14. Dispositif selon l'une quelconque des revendications 7 à 13, **caractérisé en ce que** la bobine émettrice et (ou) la bobine réceptrice sont disposées séparément chacune dans un boîtier métallique qui est au moins partiellement non ferromagnétique.

15. Dispositif selon l'une quelconque des revendications 7 à 14, **caractérisé en ce que** les bobines émettrice et réceptrice sont disposées dans un boîtier métallique commun, qui est au moins partiellement non ferromagnétique.

16. Dispositif selon l'une quelconque des revendications 7 à 15, **caractérisé en ce que** la bobine émettrice et le bobine réceptrice sont écartées dans le sens axial et séparées l'une de l'autre par une cloison métallique, et soit les deux bobines sont disposées dans un boîtier commun, soit chaque bobine est disposée dans un boîtier séparé, le ou les boîtiers se composant de matériau métallique et le matériau métallique étant au moins par endroits non ferromagnétique.

17. Dispositif selon l'une quelconque des revendications 7 à 16, **caractérisé en ce que** les bobines émettrice et réceptrice sont intégrées dans au moins une partie d'un canal de sortie d'un récipient métallurgique.

18. Procédé selon l'une ou l'ensemble des revendications 1 à 6, **caractérisé en ce qu'**un dispositif selon l'une quelconque des revendications 7 à 17 est employé pour déclencher un signal d'avertissement et (ou) un signal de commande pour déclencher un dispositif de régulation du débit et (ou) un dispositif pour la modification de l'écoulement de la masse métallique en fusion si des impuretés discrètes et (ou) continues sont détectées.
